(19) Europäisches Patentamt
European Patent Office
Office européen des brevets

(11) Veröffentlichungsnummer: **0 057 847**
**B1**

# (12) EUROPÄISCHE PATENTSCHRIFT

(45) Veröffentlichungstag der Patentschrift: **27.06.84**

(51) Int. Cl.³: **C 07 D 493/04** // A61K31/34, (C07D493/04, 307/00, 307/00)

(21) Anmeldenummer: **82100527.9**

(22) Anmeldetag: **26.01.82**

(54) **Verfahren zur Herstellung von Isosorbid-5-nitrat.**

(30) Priorität: **29.01.81 DE 3102947**

(43) Veröffentlichungstag der Anmeldung: **18.08.82 Patentblatt 82/33**

(45) Bekanntmachung des Hinweises auf die Patenterteilung: **27.06.84 Patentblatt 84/26**

(84) Benannte Vertragsstaaten: **AT BE CH DE FR GB IT LI LU NL**

(56) Entgegenhaltungen:
**EP - A - 0 045 076**
**DE - A - 2 751 934**
**DE - A - 2 903 927**

(73) Patentinhaber: **Heinrich Mack Nachf., Postfach 140, D-7918 Illertissen (DE)**

(72) Erfinder: **Stoss, Peter, Dr. Dipl.-Chem., Mozartstrasse 15, D-7918 Illertissen (DE)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

EP 0 057 847 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein neues, vorteilhaftes Verfahren zur Herstellung von Isosorbid-5-nitrat (1,4-3,6-Dianhydro-glucitol-5-nitrat) der Formel I

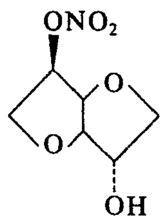

(I)

Isosorbid-5-nitrat stellt eine seit längerem bekannte Verbindung dar, deren therapeutische Einsatzmöglichkeit in jüngster Zeit verstärkt diskutiert wird. Ausgangspunkt dieses Interesses war die Beobachtung, daß Isosorbid-2,5-dinitrat (ISD), eine zur Behandlung konorarer Erkrankungen gut eingeführte Substanz, im Organismus einer raschen Metabolisierung unterliegt. Dabei treten unter anderem Isosorbid-2-nitrat (2-ISM) und Isosorbid-5-nitrat (5-ISM) als Metaboliten auf. [S. F. Sisenwine und H. W. Ruelius, J. Pharmacol. Exp. Ther. 176, 269 (1971); W. H. Down et al., J. Pharm. Sci. 63, 1147 (1974); L. F. Chausseaud et al., Eur. J. Clin. Pharmacol. 8, 157 (1975)].

Inzwischen konnte nachgewiesen werden, daß den Metaboliten 2-ISM und 5-ISM eine prinzipiell gleichartige Wirkung zukommt, wie der Muttersubstanz ISD [R. L. Wendt, J. Pharmacol. Exp. Ther. 180, 732 (1972); M. G. Bogaert et al., Naunyn-Schmiedebergs Arch. Pharmacol 75, 339 (1972); M. Stauch et al., Verh. Dtsch. Ges. Kreislaufforsch. 41, 182 (1975); D. Michel, Herz-Kreislauf 8, 444 (1976)].

Wie weiter festgestellt wurde, zeichnen sich 2-ISM und 5-ISM, hinsichtlich verschiedener therapeutisch wichtiger Parameter, wie Resorptionsverhalten, Halbwertszeit, Toxizität, orale Verabreichbarkeit und dergl. gegenüber ISD vorteilhaft aus. Es kann deshalb angebracht sein, anstelle von ISD, die Mononitrate, insbesondere Isosorbid-5-nitrat zur Behandlung von Herzerkrankungen, z. B. Angina Pectoris, zu verabreichen. Diese Anwendung setzt jedoch eine wirtschaftliche, technische Zugänglichkeit der reinen Substanzen voraus.

Die bisher bekanntgewordenen Synthesen für 5-ISM können hinsichtlich technischer Durchführbarkeit, Ausbeute und Kosten nicht befriedigen. Folgende Methoden sind beschrieben:

Isosorbid wird der direkten Nitrierung unterworfen, wobei ein Gemisch aus ISD, 2-ISM, 5-ISM und unverändertem Isosorbid, mit wechselnder Zusammensetzung, anfällt. Dieses Gemisch muß durch aufwendige und großtechnisch nicht durchführbare chromatographische Trennverfahren in die einzelnen Bestandteile zerlegt werden. Die dabei erzielte Ausbeute an 5-ISM ist so gering und die Isolierungsmethode so zeitraubend und teuer, daß diesem Weg keine praktische Bedeutung zukommt [I. G. Csizmadia und L. D. Hayward, Photochem. Photobiol. 4, 657 (1965)].

Auch ein weiteres Verfahren, wonach Isosorbid zunächst in das 2,5-Dinitrat überführt und dieses nachfolgend partiell hydrolysiert wird, ergibt wiederum die schon erwähnten Gemische aus ISD, 2-ISM, 5-ISM und Isosorbid, deren Trennung und Isolierung nicht auf wirtschaftlich vertretbare Weise durchführbar ist. [M. Anteunis et al., Org. Magnet. Resonance 3, 693 (1971)].

In der DE-OS 2 751 934 und in der korrespondierenden US-PS 4 065 488 ist folgendes Verfahren beschrieben:

Isosorbid wird mit einem Säurechlorid oder -anhydrid acyliert, wobei man eine Mischung aus Isosorbid-2-acylat, -5-acylat, -2,5-diacylat und Isosorbid erhält. Daraus wird Isosorbid extrahiert, um in der nachfolgenden Nitrierungsstufe die Bildung des als explosionsgefährlich bekannten ISD zu verhindern. Das verbleibende Gemisch aus Isosorbid-2-acylat, -5-acylat und -2,5-diacylat wird mit Salpetersäure nitriert unter Entstehung einer Mischung von Isosorbid-5-acylat-2-nitrat, -2-acylat-5-nitrat und -2,5-diacylat. Durch selektive Hydrolyse erhält man daraus ein Gemisch aus Isosorbid-2-nitrat, Isosorbid-5-nitrat und Isosorbid. Letzteres muß wiederum durch Extraktion entfernt werden, ehe aus dem verbleibenden Rückstand durch Auskristallisieren aus geeigneten Lösungsmitteln Isosorbid-2-nitrat als Hauptbestandteil isoliert wird. Isosorbid-5-nitrat verbleibt dabei in den Mutterlaugen. Eine Lehre zur Isolierung wird nicht gegeben.

Alle vorgenannten Verfahren sind dadurch gekennzeichnet, daß keine selektive Herstellung von Isosorbid-5-nitrat möglich ist, sondern stets Gemische anfallen, die nachfolgend durch geeignete Trennverfahren in die einzelnen Bestandteile zerlegt werden müssen. Diese Vorgehensweise ist aufwendig und teuer.

Sie gestattet die Isolierung des gewünschten Endproduktes jeweils nur in geringer Ausbeute und läßt damit eine wirtschaftliche Herstellung nicht zu.

Kürzlich wurde in der DE-AS 2 903 927 das erste Verfahren zur selektiven Herstellung von Isosorbid-5-nitrat bekannt gemacht. Danach wird Isomannid mittels des Halogenids oder Anhydrids einer geeigneten Sulfonsäure bzw. Carbonsäure in den entsprechenden Isomannid-2-ester verwandelt. Dessen

Umsetzung mit dem Alkali- oder Ammoniumsalz einer Benzoesäure oder mit Tetrabutylammoniumacetat oder Tetrabutylammoniumformiat liefert einen Isosorbid-2-ester. Dieser wird nachfolgend in bekannter Weise mit Salpetersäure verestert und das gebildete Isosorbid-2-ester-5-nitrat, unter Abspaltung der 2-Estergruppe, partiell hydrolysiert, wobei man Isosorbid-5-nitrat erhält. Dieses Verfahren besitzt gegenüber den früher bekannten zweifellos den Vorteil, in einer eindeutigen Reaktionsfolge zu nur einem definierten Produkt zu führen. Hinsichtlich Aufwand und Wirtschaftlichkeit läßt es jedoch noch zu wünschen übrig. So ist der als Ausgangsstoff eingesetzte Isomannid derzeit wesentlich schwerer zugänglich und auch erheblich teurer als Isosorbid. Weiterhin besteht das Verfahren aus 4 Stufen und erfordert deshalb einen hohen apparativen und personellen Zeitaufwand. Schließlich liegen die Gesamtausbeuten über alle 4 Verfahrensschritte unter 50% der Theorie.

Es besteht deshalb nach wie vor ein Bedürfnis nach Herstellungsverfahren für Isosorbid-5-nitrat, die von preiswerten Ausgangsprodukten ausgehen und in wenigen Verfahrensschritten mit besseren Ausbeuten die gewünschte Verbindung liefern.

Ein derartiges vorteilhaftes Verfahren zur Herstellung von Isosorbid-5-nitrat ist Gegenstand dieser Erfindung.

Bekanntlich kommen für die Acylierung von Isosorbid im Wesentlichen folgende Verfahren in Betracht:

1. Veresterung von Isosorbid mit Säuren
2. Umesterung von Isosorbid mit Säure-estern
3. Acylierung von Isosorbid mit Acylhalogeniden
4. Acylierung von Isosorbid mit Anhydriden.

Als Diol bietet Isosorbid zwei Angriffspunkte. Demgemäß erhält man bei Acylierungsversuchen stets Gemische aus Isosorbid, Isosorbid-2-acylat, Isosorbid-5-acylat und Isosorbid-2,5-diacylat, mit wechselnden Anteilen der einzelnen Komponenten. Die Zusammensetzung des Produktgemisches schwankt dabei in Abhängigkeit von der Herstellungsmethode und den Reaktionsbedingungen. Systematische Untersuchungen darüber liegen bisher noch nicht vor. Lediglich über vereinzelte Beobachtungen wurde berichtet.

So wird z. B. in der schon erwähnten DE-OS 2 751 934 ein durch Acylierung von Isosorbid mit Säureanhydriden, in Gegenwart saurer Katalysatoren, erhaltenes Gemisch der vier möglichen Bestandteile, mit nicht näher mitgeteilter Zusammensetzung, beschrieben.

K. W. Buck et al., Carbohydrate Res. 2, 122 (1966) erhielten bei der Umsetzung von Isosorbid mit 1 mol Acetanhydrid in Pyridin, neben Isosorbid-2,5-diacetat als Hauptprodukt, 2- und 5-Monoacetat im Verhältnis von etwa 1,7 : 1. In Gegenwart von Pyridin-hydrochlorid konnte dagegen eine Produktverteilung 2-Acetat/5-Acetat von etwa 1 : 3,6 erhalten werden, jedoch insgesamt wieder nur in untergeordneter Menge, neben 2,5-Diacetat. Gleichzeitig wiesen diese Autoren darauf hin, daß bei keinem der beiden Isosorbidmonoacetate, unter dem Einfluß von Pyridin , ohne oder mit Zusatz von Pyridin-hydrochlorid, eine Acylgruppenwanderung stattfand.

Es war deshalb überraschend und nicht vorhersehbar, daß unter ähnlichen Bedingungen, wie in der obigen Literatur zitiert, eine Acylgruppenwanderung am Isosorbid-Molekül erreicht werden konnte. Mit Hilfe des erfindungsgemäßen Verfahrens gelingt es, eine katalytische Umacylierung zu bewirken, wobei, unabhängig von der ursprünglichen Produktverteilung, stets ein Gemisch mit annähernd konstanter Produktverteilung entsteht, in dem Isosorbid-2-acylat überwiegt. Das der Umacylierung zugängliche Ausgangsprodukt kann bestehen aus einem auf bekannte Weise erhaltenen Acylierungsgemisch, mit wechselnden Anteilen von Isosorbid, Isosorbid-2-acylat, Isosorbid-5-acylat und Isosorbid-2,5-diacylat, wobei die Anteile der einzelnen Komponenten in weiten Bereichen schwanken können, oder aus einer äquimolaren Mischung von Isosorbid und Isosorbid-2,5-diacylat, oder aus reinem Isosorbid-5-acylat. Trennt man nun aus diesem, durch Umacylierung erhaltenen Gemisch, mit annähernd konstanter Produktzusammensetzung, auf destillativem Weg den Anteil des Isosorbid-2-acylats kontinuierlich ab, so verschiebt sich das Gleichgewicht im Destillationsgefäß unter katalytischem Einfluß ständig wieder zugunsten des Isosorbid-2-acylats, so daß dieses schließlich in hoher Ausbeute und guter Reinheit isoliert werden kann. Zweckmäßigerweise erfolgt die destillative Abtrennung des Isosorbid-2-acylats mittels einer Kolonne, die eine ausreichende Anzahl theoretischer Böden besitzt. Dadurch gelingt es, das gewünschte Isosorbid-2-acylat im Destillat derart anzureichern, daß es entweder direkt oder gegebenenfalls nach einem weiteren Reinigungsschritt, beispielsweise durch Umkristallisation oder Extraktion, in einer für die weitere Umsetzung genügend reinen Form anfällt. Die weitere Reinigung kann auch durch erneute Destillation vorgenommen werden.

Unter Acylgruppen, die der erfindungsgemäßen katalytischen Umacylierung unterliegen, sind geradkettige oder verzweigte aliphatische Carbonsäurereste mit 1 bis 6 Kohlenstoffatomen zu verstehen, beispielsweise Formyl, Acetyl, Propionyl, Butyryl, Valeryl, Capronyl, Isobutyryl, Isovaleryl und Pivaloyl.

Die Umacylierung wird bewirkt durch eine große Anzahl verschiedener Katalysatoren: Sulfonsäure, anorganische Säuren, Ammonium- und Metallsalze der Kohlensäure sowie Ammonium- und Metallsalze geradkettiger oder verzweigter Niederalkansäuren mit 1 bis 6 Kohlenstoffatomen, (die zur Salzbildung herangezogenen Metalle können sowohl der Gruppe der Alkalien und Erdalkalien, wie auch

anderen Gruppen des Periodensystems angehören), Alkali- und Erdalkali-hydride und -alkoholate, Metalloxide und Hydroxide, Triethylamin, 4-Dimethylamino-pyridin, Triphenylamin sowie Phasentransferkatalysatoren aus der Gruppe der quarternären Ammonium- und Phosphoniumverbindungen.

Beispielhaft werden aufgeführt:

Methansulfonsäure, Benzolsulfonsäure, p-Toluol-sulfonsäure, Phosphorsäure, Natriumacetat, Kaliumacetat, Ammoniumacetat, Calciumacetat, Bariumacetat, Bleiacetat, Cobaltacetat, Natriumpropionat, Ammoniumbutyrat, Natriumisobutyrat, Kaliumcapronat, Kaliumcarbonat, Natriumcarbonat, Calciumcarbonat, Natriumhydrid, Kaliumhydrid, Calciumhydrid, Natriummethylat, Kalium-tert.-butylat, Aluminiumoxid, Calciumoxid, Natriumhydroxid, Kaliumhydroxid, Calciumhydroxid, Triethylamin, 4-Dimethylamino-pyridin, Triphenylamin, Cetyl-trimethylammoniumbromid, Tetrabutylammoniumhydrogensulfat, Benzyl-triethylammonium-chlorid, Tetraethylammoniumhydroxid und Benzyl-triphenylphosphonium-bromid.

Das erfindungsgemäße Verfahren wird wie folgt durchgeführt:

Ein auf bekannte Weise erhaltenes Acylierungsgemisch aus Isosorbid, Isosorbid-2-acylat, Isosorbid-5-acylat und Isosorbid-2,5-diacylat mit wechselnder Zusammensetzung, oder eine äquimolare Mischung von Isosorbid und Isosorbid-2,5-diacylat, oder reines Isosorbid-5-acylat, wird mit 0,001 bis 0,01 Moläquivalenten des Katalysators versetzt und in einem Destillationsgefäß mit aufgesetzter Kolonne einer Destillation unterworfen. Dabei destilliert bevorzugt Isosorbid-2-acylat ab. Im Destillationssumpf findet laufend eine Umacylierung statt, so daß ständig das aus dem Gleichgewicht entfernte Isosorbid-2-acylat nachgebildet wird und sich im Destillat anreichert. Man erhält auf diese Weise, abhängig von der Art des Katalysators, der Güte der verwendeten Kolonne, dem Rücklaufverhältnis und dergl., Isosorbid-2-acylat in etwa 85- bis 95prozentiger Reinheit und mit nahezu quantitativer Ausbeute.

Zweckmäßigerweise wird dieses so erhaltene Isosorbid-2-acylat einer zusätzlichen Reinigung durch Umkristallisation und/oder Extraktion unterworfen, wodurch man Isosorbid-2-acylat mit über 99%iger Reinheit erhält. Die bei dieser Reinigung anfallenden Mutterlaugen, die neben wenig Isosorbid-2-acylat vorwiegend Isosorbid, Isosorbid-5-acylat und Isosorbid-2,5-diacylat enthalten, können erneut der Umacylierung unterworfen, beispielsweise dem Destillationsgefäß wieder zugeführt oder einem neuen Ansatz zugesetzt werden, so daß auf diese Weise eine nahezu quantitative Umsetzung, in bezug auf Isosorbid-2-acylat, erreichbar ist.

Die Fortführung des Verfahrens besteht darin, das auf diesem wirtschaftlichen Weg erstmals in beliebig großen Mengen zugängliche, sehr reine Isosorbid-2-acylat, in an sich bekannter Weise mit Salpetersäure zu verestern, wobei Isosorbid-2-acylat-5-nitrat erhalten wird, welches durch keinerlei Nebenprodukte verunreinigt ist. In der nächsten Stufe wird schließlich, durch alkalische Hydrolyse bzw. Umesterung, in an sich bekannter Weise, z. B. in Anlehnung an die DE-OS 2 751 934, mittels Natrium- oder Kaliumhydroxid in organischem oder wäßrig-alkoholischem Milieu, oder in einem niederen Alkohol in Anwesenheit eines Alkalialkoholats, die 2-Acylgruppe selektiv abgespalten und damit reines Isosorbid-5-nitrat erhalten.

Zur Lösung der zuvor angegebenen Aufgabe der Herstellung von Isosorbid-5-nitrat auf einfachem Wege und aus leicht zugänglichen Ausgangsmaterialien dient daher das Verfahren, wie es im Patentanspruch 1 näher beschrieben ist. Vorteilhafte Ausführungsformen dieses Verfahrens sind in den Patentansprüchen 2 und 3 näher angegeben.

Insgesamt wird das erfindungsgemäße Verfahren daher wie folgt durchgeführt:

In einer ersten Stufe wird durch katalytische Umacylierung eines auf geeignete Weise bereiteten Gemisches aus Isosorbid, Isosorbid-5-acylat, Isosorbid-2-acylat und Isosorbid-2,5-diacylat oder von einer äquimolaren Mischung von Isosorbid und Isosorbid-2,5-diacylat oder von reinem Isosorbid-5-acylat unter kontinuierlichem Abdestillieren des im Ausgangsgemisch bereits vorliegenden oder laufend nachgebildeten Isosorbid-2-acylates möglichst reines Isosorbid-2-acylat gewonnen, das in einer zweiten Stufe in an sich bekannter Weise mit Salpetersäure zur Isosorbid-2-acylat-5-nitrat verestert wird, das dann in einer dritten Stufe in an sich bekannter Weise partiell zu reinem Isosorbid-5-nitrat hydrolisiert wird.

Vorteilhafterweise wird das am Ende der ersten Stufe gewonnene Isosorbid-2-acylat vor dem Einführen in die zweite Stufe noch einer weiteren Reinigungsstufe, beispielsweise durch erneute Destillation, Extraktion oder insbesondere Umkristallisation unterworfen.

Der Vorzug des erfindungsgemäßen Verfahrens gegenüber den eingangs erwähnten, bekannten Verfahren, insbesondere gegenüber dem bisher als am vorteilhaftesten anzusehenden der DE-AS 2 903 927, liegt darin, daß als Ausgangsprodukt der leichter zugängliche und erheblich billigere Isosorbid eingesetzt werden kann. Weiterhin umfaßt das Verfahren in der Grundausführungsform lediglich 3 Stufen, die technisch leicht durchzuführen sind. Und schließlich liegen die Ausbeuten über alle Stufen wesentlich über denen der vorbeschriebenen Verfahren.

Die folgenden Beispiele dienen zur Erläuterung der Erfindung:

## Beispiel 1

146 g (1 mol) Isosorbid und 230 g (1 mol) Isosorbid-2,5-diacetat werden mit 2 g Natriummethylat versetzt und im Vakuum über eine Kolonne destilliert. Dabei erhält man 360 g eines bei 98—100°C/0,1 mbar übergehenden Destillates, das in der Vorlage alsbald auskristallisiert. Die Substanz besteht zum überwiegenden Teil (85—95%) aus Isosorbid-2-acetat, neben wenig Isosorbid, Isosorbid-2,5-diacetat und Spuren von Isosorbid-5-acetat. Durch einmaliges Umkristallisieren, z. B. aus Aceton, erhält man Isosorbid-2-acetat in über 99proz. Reinheit; Schmp. 80°C. Die beim Umkristallisieren anfallende Mutterlauge wird, nach Entfernen des Lösungsmittels, erneut dem Umacylierungsprozeß unterworfen bzw. dem nächsten Ansatz zugesetzt, wodurch eine nahezu quantitative Bildung von Isosorbid-2-acetat erreichbar ist.

## Beispiel 2

146 g (1 mol) Isosorbid, 60 g (1 mol) Essigsäure und 1 g p-Toluolsulfonsäure werden in 250 ml Benzol solange am Wasserabscheider unter Rückfluß erhitzt, bis sich die theoretische Menge an Wasser (18 ml) abgeschieden hat. Das erhaltene Gemisch, bestehend aus etwa gleichen Anteilen von Isosorbid, Isosorbid-2-acetat, Isosorbid-5-acetat und Isosorbid-2,5-diacetat wird unter Zusatz von 2 g Kaliumcarbonat der Umacylierung unterworfen und destilliert. Bei 98—100°C/0,1 mbar gehen 180 g eines Destillats über, das kristallin erstarrt und in seiner Zusammensetzung dem in Beispiel 1 beschriebenen entspricht.

## Beispiel 3

188 g Isosorbid-5-acetat (1 mol) werden nach Zusatz von 1 g Natriumhydrid analog Beispiel 1 behandelt, wobei 180 g eines Destillates mit vergleichbarer Produktzusammensetzung resultieren.

## Beispiel 4

Zu 146 g Isosorbid (1 mol) und 1 g Natriumacetat läßt man bei ca. 100°C 102 g (1 mol) Essigsäureanhydrid fließen. Man erhitzt noch eine halbe Stunde unter Rückfluß und entfernt anschließend im Wasserstrahlvakuum bis zu einer Sumpftemperatur von ca. 140°C die entstandene Essigsäure. Eine Analyse des Rückstandes zeigt Isosorbid, Isosorbid-2-acetat, Isosorbid-5-acetat und Isosorbid-2,5-diacetat in etwa gleichen Anteilen. Bei der nachfolgenden Umacylierung und Destillation, wie in Beispiel 1 angegeben, entstehen 182 g rohes Isosorbid-2-acetat. Nach dem Umkristallisieren aus 200 ml Aceton erhält man 92 g reines Isosorbid-2-acetat vom Schmp. 80°C. Der nach dem Einengen des Filtrats verbleibende Rückstand (90 g) wird nach Zugabe von 0,5 g Natriumacetat erneut der Umacylierung zugeführt, wobei wiederum in nahezu quantitativer Ausbeute rohes Isosorbid-2-acetat entsteht. Daraus werden wie oben beschrieben, 43 g reines Isosorbid-2-acetat und 46 g Rückstand gewonnen. Aus letzterem lassen sich durch eine weitere Destillation, in Gegenwart von Natriumacetat, 44 g rohes Isosorbid-2-acetat herstellen, woraus durch Umkristallisation 26 g reines Isosorbid-2-acetat isoliert werden. Auf diese Weise erhält man somit insgesamt 161 g reines Isosorbid-2-acetat, entsprechend 83% der Theorie und 18 g Rückstand, der weiteren Umacylierungen zugänglich ist. Durch Wiederholung der vorstehenden Arbeitsweise bzw. durch Zugabe des jeweiligen Rückstandes zum nächsten Acylierungsansatz ist eine nahezu quantitative Ausbeute an reinem Isosorbid-2-acetat zu erreichen.

## Beispiel 5

Setzt man anstelle der im Beispiel 2 beschriebenen Essigsäure, oder anstelle des im Beispiel 4 beschriebenen Essigsäureanhydrids, die entsprechenden geradkettigen oder verzweigten homologen Carbonsäuren oder Carbonsäure-anhydride ein, oder führt man die Acylierung des Isosorbids mittels der entsprechenden Carbonsäure-halogenide oder -ester durch, oder geht man, wie im Beispiel 1 beschrieben, von einer Mischung aus Isosorbid und dem entsprechenden Isosorbid-2,5-diacylat, oder, wie im Beispiel 3 beschrieben, von reinem Isosorbid-5-acylat aus, so erhält man nach der katalytischen Umacylierung, gemäß den vorstehenden Beispielen, die nachfolgend aufgeführten Isosorbid-2-acylate in vergleichbarer Qualität und Ausbeute:

| | |
|---|---|
| 2-Formyl-isosorbid | Schmp. 62°C |
| 2-Propionyl-isosorbid | Schmp. 47°C |
| 2-Butyryl-isosorbid | Schmp. 51—52°C |
| 2-Valeryl-isosorbid | Schmp. 39—40°C |

| | |
|---|---|
| 2-Capronyl-isosorbid | Schmp. 57° C |
| 2-Isobutyryl-isosorbid | ölig |
| 2-Isovaleryl-isosorbid | ölig |
| 2-Pivaloyl-isosorbid | Schmp. 58° C |

Beispiel 6

Die katalytische Umacylierung tritt auch ein beim Ersatz der in den Beispielen 1 bis 4 beschriebenen durch die nachfolgend aufgeführten Katalysatoren:

Methansulfonsäure, Benzolsulfonsäure, p-Toluol-sulfonsäure, Phosphorsäure, Ammoniumacetat, Kaliumacetat, Calciumacetat, Bariumacetat, Nickelacetat, Cobaltacetat, Bleiacetat, Natriumpropionat, Ammoniumbutyrat, Kaliumisobutyrat, Natriumcapronat, Natriumcarbonat, Calciumcarbonat, Kaliumhydrid, Calciumhydrid, Natrium-ethylat, Kalium-tert.butylat, Aluminiumoxid, Calciumoxid, Natriumhydroxid, Kaliumhydroxid, Calciumhydroxid, Triethylamin, 4-Dimethylamino-pyridin, Triphenylamin, Cetyl-trimethylammoniumbromid, Tetrabutylammoniumhydrogensulfat, Benzyl-triethylammoniumchlorid, Tetraethylammoniumhydroxid, Benzyl-triphenylphosphoniumbromid.

So erhält man aus 146 g (1 mol) Isosorbid und 230 g (1 mol) Isosorbid-2,5-diacetat, unter Zusatz von 1—2 g eines der aufgeführten Katalysatoren und Behandeln des Gemisches wie in Beispiel 1 beschrieben, 340—365 g Destillat, das kristallin erstarrt und die in Beispiel 1 angegebene Zusammensetzung aufweist.

Beispiel 7

Zu einer aus 130 g 65proz. Salpetersäure und 400 ml Essigsäureanhydrid unter Kühlung bereiteten Nitriermischung gibt man portionsweise, unter Rühren bei 5 bis 10° C, 188 g reines Isosorbid-2-acetat. Nachdem man noch 1 Stunde bei der gleichen Temperatur gerührt hat, gießt man in 1 l Wasser und extrahiert 2mal mit je 300 ml Methylenchlorid. Die vereinigten organischen Phasen werden vorsichtig mit verdünnter wäßriger Natriumcarbonatlösung gewaschen und danach vom Lösungsmittel befreit. Den Rückstand nimmt man in 500 ml Methanol auf, versetzt mit 5 ml 35proz. methanolischer Natriummethylat-Lösung und beläßt das Gemisch bei Raumtemperatur, bis dünnschichtchromatografisch eine vollständige Umsetzung festzustellen ist (ca. 1 Stunde). Nach der Neutralisation mit Essigsäure wird im Vakuum zur Trockne eingedampft und der Rückstand aus 300 ml Wasser umkristallisiert.

Ausbeute: 160 g (85% d. Theorie) Isosorbid-5-nitrat, Schmp. 93° C.

Beispiel 8

Bei gleicher Arbeitsweise wie im vorstehenden Beispiel 7 beschrieben, jedoch unter Ersatz des reinen Isosorbid-2-acetats durch 188 g rohes Isosorbid-2-acetat, wie es bei der Destillation nach den Beispielen 1 bis 4 anfällt, nimmt man den nach Neutralisation und Eindampfen der methanolischen Lösung zur Trockne verbleibenden Rückstand in 500 ml Wasser bei 40° C auf und extrahiert mit 100 ml Toluol/Petroläther (1 : 1). Der organische Extrakt enthält geringe Mengen von Isosorbid-2,5-dinitrat. Die Wasserphase wird auf 400 ml eingeengt, auf 0° C abgekühlt und das auskristallisierte Isosorbid-5-nitrat abgesaugt. Man erhält so 135 g (71% der Theorie) vom Schmp. 93° C.

Beispiel 9

Anstelle des in den Beispielen 7 und 8 eingesetzten Isosorbid-2-acetats werden die nach Beispiel 5 hergestellten geradkettigen oder verzweigten homologen Isosorbid-2-acylate analog der Veresterung mit Salpetersäure und nachfolgenden partiellen Hydrolyse unterworfen, wobei jeweils Isosorbid-5-nitrat, vom Schmp. 93° C, in Ausbeuten zwischen 70 und 85% erhalten wird.

**Patentansprüche**

1. Verfahren zur Herstellung von Isosorbid-5-nitrat durch Herstellung von Isosorbid-2-acylat, Veresterung dieses Isosorbid-2-acylates mit Salpetersäure zu Isosorbid-2-acylat-5-nitrat und anschließende partielle Hydrolyse dieses Isosorbid-2-acylat-5-nitrates zu Isosorbid-5-nitrat, dadurch gekennzeichnet, daß man

a) ein Acylierungsgemisch von Isosorbid, das wechselnde Anteile von Isosorbid, Isosorbid-2-acylat, Isosorbid-5-acylat und/oder Isosorbid-2,5-diacylat enthält, oder reines Isosorbid-5-acylat oder ein äquimolares Gemisch von Isosorbid-2,5-diacylat und Isosorbid, in Anwesenheit eines Umesterungskatalysators aus der Gruppe bestehend aus Sulfonsäuren, anorganischen Säuren, Metall- oder Ammoniumkarbonaten, Metall- oder Ammoniumsalzen von geradkettigen oder verzweigten Niederalkansäuren mit 1 bis 6 Kohlenstoffatomen, Alkali- oder Erdalkalihydriden oder -alkoholaten, Metalloxiden oder -hydroxiden, Triethylamin, 4-Dimethylamino-pyridin, Triphenylamin und Phasentransferkatalysatoren in Form von quaternären Ammonium- oder Phosphoniumverbindungen einer Umacylierungsreaktion unterwirft und das vorliegende Isosorbid-2-acylat aus dem Reaktionsgemisch durch fraktionierte Destillation entfernt,
b) das abgetrennte Isosorbid-2-acylat gegebenenfalls einer weiteren Reinigungsstufe unterwirft,
c) das erhaltene Isosorbid-2-acylat mit Salpetersäure verestert und
d) das erhaltene Isosorbid-2-acylat-5-nitrat partiell hydrolysiert.

2. Verfahren nach Anspruch 1, dadurch gekennzeichnet, daß man als Isosorbid-acylat Isosorbidacetat verwendet.

3. Verfahren nach Anspruch 1, dadurch gekennzeichnet, daß man in Stufe b) das abgetrennte Isosorbid-2-acylat durch Umkristallisation oder Extraktion reinigt.

## Claims

1. Process for the preparation of isosorbid-5-nitrate by preparation of isosorbid-2-acylate, esterification of this isosorbid-2-acylate with nitric acid to yield isosorbid-2-acylate-5-nitrate and subsequent partial hydrolysis of this isosorbid-2-acylate-5-nitrate to yield isosorbid-5-nitrate, characterized in that

a) an acylation mixture of isosorbid, containing changing proportions of isosorbid, isosorbid-2-acylate, isosorbid-5-acylate and/or isosorbid-2,5-diacylate, or pure isosorbid-5-acylate or an equimolar mixture of isosorbid-2,5-diacylate and isosorbid is subjected to a re-acylation reaction in the presence of a re-esterification catalyst selected from the group consisting of sulfonic acids, inorganic acids, metal- or ammoniumcarbonates, metal or ammonium salts of linear or branched lower alkane acids with 1 to 6 carbon atoms, alkali or alkaline earth hydrides or alkali or alkaline earth alcoholates, metal oxides or metal hydroxides, triethylamine, 4-dimethylamino-pyridine, triphenylamine and phasetransfercatalysts in form of quaternary ammonium or phosphonium compounds, and the isosorbid-2-acylate present is removed from the reaction mixture by fractionate distillation,
b) the separated isosorbid-2-acylate is subjected, if desired, to a further purification step,
c) the isosorbid-2-acylate abtained is esterified with nitric acid and
d) the isosorbid-2-acylate-5-nitrate obtained is partially hydrolized.

2. Process according to claim 1 characterized in that as isosorbid-acylate isosorbid-acetate is used.

3. Process according to claim 1 characterized, in that in step b) the separated isosorbid-2-acylate is purified by recristallisation or extraction.

## Revendications

1. Procédé de production de 5-nitrate, d'isosorbide par formation de 2-acylate d'isosorbide, estérification de ce 2-acylate d'isosorbide avec de l'acide nitrique en 5-nitrate de 2-acylate d'isosorbide et hydrolyse parcielle subséquente du 5-nitrate de 2-acylate d'isosorbide en 5-nitrate d'isosorbide, caractérisé en ce que

a) on soumet à une réaction de transacylation un mélange acylé d'isosorbide, qui contient des proportions variables d'isosorbide, de 2-acylate d'isosorbide, de 5-acylate d'isosorbide et/ou de 2,5-diacylate d'isosorbide, ou du 5-acylate d'isosorbide pur ou un mélange équimolaire de 2,5-diacylate d'isosorbide et d'isosorbide, en présence d'un catalyseur de transestérification choisi dans le groupe comprenant des acides sulfoniques, des acides inorganiques, des carbonates de métaux ou d'ammonium, des sels de métaux ou d'ammonium d'acides alcanoïques inférieurs à chaîne droite ou ramifiés ayant 1 à 6 atomes de carbone, des hydrures ou alcoolates de métaux alcaline ou alcalino-terreux, des oxydes ou hydroxydes métalliques, la triéthylamine, la 4-diméthylaminopyridine, la triphénylamine et des catalyseurs de transfert de phase sous forme de composés d'ammonium ou de phosphonium quaternaires et on élimine du mélange réactionnel le 2-acylate d'isosorbide présent par distillation fractionnée,

b) on soumet, le cas échéant, le 2-acylate d'isosorbide séparé à une autre opération de purification,
c) on estérifie à l'acide nitrique le 2-acylate d'isosorbide obtenu et
d) on hydrolyse partiellement le 5-nitrate de 2-acylate d'isosorbide obtenu.

2. Procédé suivant la revendication 1, caractérisé en ce qu'on utilise comme acylate d'isosorbide l'acétate d'isosorbide.

3. Procédé suivant la revendication 1, caractérisé en ce qu'on purifie par recristallisation ou par extraction dans l'étape b) le 2-acylate d'isosorbide séparé.